(19) Europäisches Patentamt
European Patent Office
Office européen des brevets

(11) **EP 3 269 850 A1**

(12) **EUROPEAN PATENT APPLICATION**
published in accordance with Art. 153(4) EPC

(43) Date of publication:
**17.01.2018 Bulletin 2018/03**

(51) Int Cl.:
*D01F 8/04* (2006.01)      *A01N 25/34* (2006.01)
*A01N 53/08* (2006.01)      *A01P 7/04* (2006.01)

(21) Application number: **16761776.0**

(22) Date of filing: **09.03.2016**

(86) International application number:
**PCT/JP2016/057317**

(87) International publication number:
**WO 2016/143809 (15.09.2016 Gazette 2016/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.03.2015 JP 2015046525**

(71) Applicant: **NBC Meshtec, Inc.**
**Hino-shi, Tokyo 191-0053 (JP)**

(72) Inventors:
• **AMEMIYA, Yosuke**
  **Hino-shi**
  **Tokyo 191-0053 (JP)**

• **MOTOJIMA, Nobukazu**
  **Hino-shi**
  **Tokyo 191-0053 (JP)**
• **NAKAYAMA, Tsuruo**
  **Hino-shi**
  **Tokyo 191-0053 (JP)**

(74) Representative: **Wynne-Jones, Lainé and James LLP**
**Essex Place**
**22 Rodney Road**
**Cheltenham**
**Gloucestershire GL50 1JJ (GB)**

(54) **INSECT REPELLENT FIBER AND INSECT REPELLENT NET USING SAME**

(57) [Problem] To exhibit an insect-repelling effect while suppressing insect repellent intake by people, etc., and to prolong said insect-repelling effect.

[Solution] This insect repellent fiber (1), which can release an insect repellent, comprises a core part (2) which contains an insect repellent (4) and which is formed from a thermoplastic resin, and a sheath part (3) which contacts the outer surface of the core part to cover the core part and which is formed from a thermoplastic resin. The crystallinity of the thermoplastic resin configuring the sheath part is less than or equal to the crystallinity of the thermoplastic resin configuring the core part.

FIG.1

**Description**

Technical Field

The present invention relates to an insect repellent fiber having a repelling effect against pest insects (insect repellent effect) and an insect repellent screen using the same.

Background Art

Conventionally, a screen door has been widely used in a house to prevent the intrusion of insects while allowing the flow of natural wind. However, when openings of the screen door are made smaller to prevent the intrusion of small insects into the house, the air permeability of the screen door is reduced. On the other hand, when the openings of the screen door are made larger to improve the air permeability of the screen door, small insects tend to easily intrude into the house.

Thus, it has been desired to develop an insect repellent fiber having a repelling effect against pest insects to produce an insect repellent screen that has openings capable of ensuring the air permeability and prevents the intrusion of insects.

As methods for solving these problems, there are proposed an insect repellent filament having a sheath-core structure in which an insect repellent active compound is mixed to a sheath part (Patent Literature 1) and an insect repellent screen which is produced by impregnat ing a lace consisted of multifilament fibers with an insect repellent and an adhesive (Patent Literature 2).

Citation List

Patent Literature

   Patent Literature 1: JP2010-13390
   Patent Literature 2: JP2001-220970

Summary of Invention

Technical Problem

In Patent Literatures 1 and 2, it is aimed to improve the insect repellent effect by including an insect repellent on the outer surface of fibers, thereby facilitating the release of the insect repellent to the outside of the fibers. However, as the release of the insect repellent is made easier, the insect repellent is more likely to be accidentally ingested by people and animals . Such a harmful effect to a living being such as human caused by ingesting the insect repellent is not considered at all in Patent Literatures 1 and 2.

Further, in Patent Literature 1, the insect repellent filament is configured to facilitate the release of the insect repellent from its outer surface. However, in this configuration, the dusts more easily adhere to the insect repellent and prevent its release as the amount of the insect repellent on the outer surface of the insect repellent filament increases. As a result, the insect repellent effect tends to decrease. On the other hand, washing the insect repellent filament with water to remove the dusts also removes the insect repellent along with the dusts, thereby making it difficult to maintain the insect repellent effect.

In Patent Literature 2, the insect repellent is fixed to the outer surface of the lace. In this configuration, the insect repellent sometimes falls off from the lace when an external force is applied to the lace. This reduces the insect repellent effect and shortens the duration of the insect repellent effect. Further, the dusts easily adhere to the insect repellent since the insect repellent is exposed on the outer surface of the lace. Thus, the insect repellent effect of the lace is easily reduced as described above. Further, removing the dusts adhering to the lace tends to remove the insect repellent with the dust, thus reducing the duration of the insect repellent effect.

Further, the excessive amount of the insect repellent on the outer surface of the fibers causes tackiness to the fibers. Thus, it is difficult to weave the insect repellent screen by using such fibers. In consideration of these points, the previously proposed insect repellent fibers are not sufficiently suitable for practical use.

The present invention has been made to solve the above-mentioned problems. An object of the present invention is to provide an insect repellent fiber, which can exhibit the insect repellent effect while suppressing insect repellent intake by a living being such as human and prolong the insect repellent effect, and an insect repellent screen using the insect repellent fiber. Solution to Problem

The gist of the present invention is as follows.

[1] An insect repellent fiber capable of releasing an insect repellent, comprising:

a core part containing the insect repellent and being formed from a thermoplastic resin; and
a sheath part being in contact with an outer surface of the core part to cover the core part and being formed from a thermoplastic resin, wherein
the thermoplastic resin configuring the sheath part has a crystallinity less than or equal to a crystallinity of the thermoplastic resin configuring the core part.

[2] The insect repellent fiber according to [1], wherein a main component of the insect repellent is a pyrethroid-based insect repellent.

[3] The insect repellent fiber according to [2], wherein the pyrethroid-based insect repellent is at least one or more kinds of permethrin and ethofenprox.

[4] The insect repellent fiber according to any one of [1] to [3], wherein the crystallinity of the thermoplastic resin configuring the sheath part is 80% or less.

[5] The insect repellent fiber according to any one of [1] to [4], wherein the sheath part has a thickness of 5 $\mu$m or more and 70 $\mu$m or less.

[6] The insect repellent fiber according to any one of [1] to [5], wherein a content of the insect repellent with respect to the insect repellent fiber is 0.1% by mass or more and 5% by mass or less.

[7] An insect repellent screen comprising the insect repellent fiber according to any one of [1] to [6].

Advantageous Effects of Invention

The present invention can provide an insect repellent fiber capable of exhibiting the insect repellent effect while suppressing insect repellent intake by a living being such as human and prolonging the insect repellent effect, and an insect repellent screen using the insect repellent fiber.

Brief Description of Drawings

FIG. 1 is a schematic view of a structure of an insect repellent fiber.
FIG. 2 is a cross-sectional view of an insect repellent fiber in which an insect repellent is unevenly distributed in a core part.

Description of Embodiments

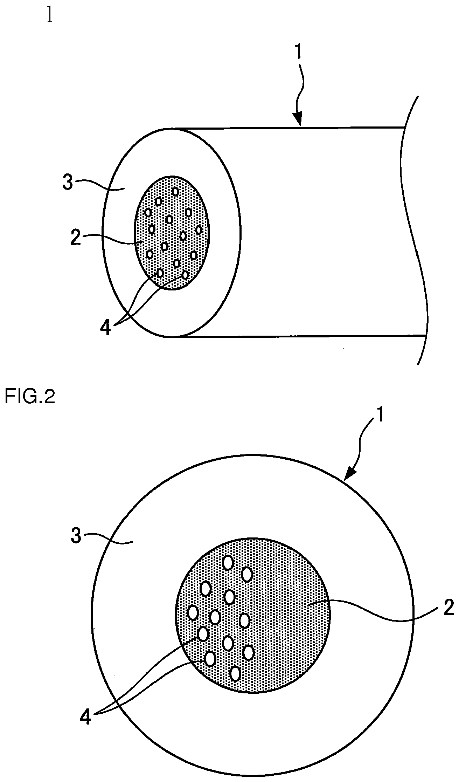

Hereinafter, one embodiment of the present invention will be described in detail with reference to FIG. 1.

The present embodiment relates to an insect repellent fiber 1 capable of releasing an insect repellent. As shown in FIG. 1, the insect repellent fiber 1 has a core part 2 and a sheath part 3. The core part 2 is formed from a thermoplastic resin and an insect repellent 4 is dispersedly retained inside the core part 2. The sheath part 3 is formed from a thermoplastic resin and is in contact with an outer surface of the core part 2 to cover the core part 2. The insect repellent 4 migrates from the core part 2 to the sheath part 3 and is then released from the outer surface of the sheath part 3 to the outside of the insect repellent fiber 1. In this configuration, the crystallinity of the thermoplastic resin configuring the sheath part 3 of the insect repellent fiber 1 is less than or equal to the crystallinity of the thermoplastic resin configuring the core part 2 of the insect repellent fiber 1.

In the insect repellent fiber 1 of the present embodiment, the thermoplastic resins configuring the core part 2 and the sheath part 3 may be appropriately selected without particular limitation, as long as they can maintain shapes of the core part 2 and the sheath part 3 and release the insect repellent 4 to the outside of the insect repellent fiber 1. Further, the thermoplastic resins configuring the core part 2 and the sheath part 3 may be the same as or different from each other. Examples of the thermoplastic resins may include polyester, polyethylene, polypropylene, polyvinyl chloride, polyethylene terephthalate, polybutylene terephthalate, polytetramethylene terephthalate, nylon, acryl, polyvinylidene fluoride, polyethylene tetrafluoroethylene, polytetrafluoroethylene, polyvinyl alcohol, Kevlar (registered trademark),polyacrylicacid, polymethylmethacrylate, rayon, cupra, Tencel (registered trade mark), polynosic, acetate, and triacetate. Of these thermoplastic resins, a crystalline thermoplastic resin is used for configuring the core part 2 and the sheath part 3 from the viewpoint of securing strength of the core part 2 and the sheath part 3. Specifically, polyethylene, polypropylene, polyethylene terephthalate, polybutylene terephthalate, nylon, polyvinylidene fluoride, and the like can be mentioned. Of these, polyethylene, polypropylene, polyethylene terephthalate, polybutylene terephthalate, and the like are more preferable as the thermoplastic resin from the viewpoint of facilitating the production of the insect repellent fiber 1 by melt spinning.

In the present embodiment, the crystallinity of the thermoplastic resin configuring the sheath part 3 is set to less than or equal to the crystallinity of the thermoplastic resin configuring the core part 2. The crystallinity of the thermoplastic resin depends on a material of the thermoplastic resin, and a heating temperature and/or stretch ratio in a heating and stretching process, which is performed after the melt spinning. Thus, by determining in advance the relationship among the crystallinity, the material of the thermoplastic resin, and the heating temperature and/or stretch ratio in the heating and stretching process performed after the melt spinning, the material of the thermoplastic resin, and the heating temperature and/or stretch ratio in the heating and stretching process performed after the melt spinning can be set so that the crystallinity of the thermoplastic resin configuring the sheath part 3 becomes less than or equal to the crystallinity of the thermoplastic resin configuring the core part 2. It is noted that, when the heating and stretching process is not performed, only the material of the thermoplastic resin needs to be set to adjust the crystallinity of the thermoplastic resin configuring the sheath part 3 to be less than or equal to the crystallinity of the thermoplastic resin configuring the core part 2. It is noted that the crystallinity of the thermoplastic resins configuring the core part 2 and the sheath part 3 can be measured, for example, by a powder X-ray diffraction method. Further, the crystallinity of the thermoplastic resin configuring the core part 2 may be considered the same as the crystallinity of the thermoplastic resin configuring a core part 2 formed in a single layer structure (i.e. fiber) under the same conditions as the core part 2 of the present embodiment.

When the crystallinity of the thermoplastic resin configuring the sheath part 3 is less than or equal to the crystallinity of the thermoplastic resin configuring the core part 2, the insect repellent 4 can easily migrate mainly in an amorphous portion of the sheath part 3. Thus, the insect repellent 4 can be released to the outside of the insect repellent fiber 1. This configuration allows for exerting the insect repellent effect. In contrast, when the crystallinity of the thermoplastic resin configuring the sheath part 3 is greater than the crystallinity of the thermoplastic resin configuring the core part 2, the insect repellent 4 hardly migrates through the sheath part 3. Therefore, the insect repellent 4 is hardly released to the outside of the insect repellent fiber 1. Thus, the insect repellent effect tends to decrease.

The crystallinity of the thermoplastic resin configuring the core part 2 is preferably 40% or more and 100% or less. The crystallinity of the thermoplastic resin configuring the sheath part 3 only needs to be less than or equal to the crystallinity of the thermoplastic resin configuring the corepart 2. Specifically, it is preferably set to less than or equal to the crystallinity of the thermoplastic resin configuring the core part 2 (40% or more and 100% or less) within the range of 40% or more and 100% or less. The crystallinity of the thermoplastic resin configuring the sheath part 3 is more preferably set to 80% or less.

The insect repellent 4 retained in the core part 2 may be appropriately selected by a person skilled in the art without particular limitation, as long as the insect repellent 4 remains in the core part 2 of the insect repellent fiber 1 during the production of the insect repellent fiber 1 by melt spinning. For example, the insect repellent 4 in use may be microencapsulated or the insect repellent 4 may be carried by a porous substance.

In the present embodiment, the insect repellent 4 to be used are preferably microencapsulated. The microencapsulated insect repellent 4 is prepared by filling the insect repellent 4 as a liquid compound in microcapsules. When the insect repellent fiber 1 is produced by the melt spinning using the crystalline thermoplastic resin, the insect repellent 4 migrates to the amorphous portion of the thermoplastic resin and some of the insect repellent 4 failing to stay in the amorphous portion bleed out to the outer surface of the insect repellent fiber 1. This causes tackiness (stickiness), which makes the weaving difficult, and requires the insect repellent 4 in an amount more than necessary during the melt spinning. When the microencapsulated insect repellent 4 is used, the insect repellent 4 as a liquid compound is retained inside the microcapsules during the melt spinning. Thus, the migration of the insect repellent 4 to the amorphous portion of the thermoplastic resin can be suppressed. This may prevent the insect repellent 4 from bleeding out to the outer surface of the insect repellent fiber 1. This configuration can prevent the occurrence of the tackiness and the use of the insect repellent 4 in an amount more than necessary.

Further, in the present embodiment, the insect repellent 4 in use are preferably carried by an inorganic compound that controls the release of the insect repellent 4. Examples of the inorganic compound that controls the release of the insect repellent 4 may include inorganic compounds having a particle shape, a fibrous shape, a plate shape, a scale shape, and a layer shape. These inorganic compounds are further preferably a porous substance to increase the surface area on which the insect repellent 4 can be carried. The use of the insect repellent 4, which are carried on the inorganic compound controlling the release of the insect repellent 4, can prevent the bleeding out of the insect repellent 4 to the outer surface of the insect repellent fiber 1. This configuration can prevent the occurrence of the tackiness and allow for releasing the insect repellent 4 in a minimum amount necessary to exert the insect repellent effect.

The insect repellent 4 of the present embodiment is preferably contained in the insect repellent fiber 1 as a liquid compound. By using the insect repellent 4 in the form of the liquid compound, it becomes possible to add the insect repellent 4 to the core part 2 at a high concentration in a stable state and easily adjust the diffusion speed of the insect repellent 4 inside the insect repellent fiber 1. Further, a main component of the insect repellent 4 is not particularly limited. However, it is preferably in a liquid state at the normal temperature. Specific examples of the main component may include: pyrethroid-based insect repellents, such aspyrethrin, cinerin, jasmolin, allethrin, resmethrin, fenvalerate, and permethrin; cyclic diene-based insect repellents, such as toxaphene and benzoepin; organic phosphorus-based insect repellents, such as malathion and fenitrothion; and carbamate-based insect repellents, such as carbaryl, methomyl, and promecarb. One kind of these insect repellents may be used alone, or two or more kinds thereof may be used in combination. Of these insect repellents, the pyrethroid-based insect repellents have excellent repelling property and immediate effectivity, show little acute toxicity, and thus are preferable. Further, among the pyrethroid-based insect repellents, permethrin and ethofenprox are capable of exhibiting the insect-repellent effect at a low concentration, allow safe use for people and animals, and thus are preferable.

In the insect repellent fiber 1 having the core part 2 and the sheath part 3 of the present embodiment, the insect repellent 4 contained in the core part 2 passes through the sheath part 3 and is released to the outside of the insect repellent fiber 1. This configuration can adjust the release of the insect repellent 4 in the sheath part 3. Thus, as compared to the configuration where the insect repellent 4 is contained in the sheath part 3, the insect repellent 4 can be more readily prevented from diffusing to the outside of the insect repellent fiber 1. Thus, the safety for people and animals can be secured and the duration of the insect repellent effect can be prolonged.

In an insect repellent fiber having a sheath-core structure in which the insect repellent 4 is contained in the sheath part 3 or an insect repellent fiber formed in a single layer containing the insect repellent 4, the tackiness occurs on the surface of the insect repellent fiber, thus making it difficult to weave the insect repellent fibers into a screen form. In contrast, in the insect repellent fiber 1 according to the present embodiment, the insect repellent 4 contained in the core part 2 passes through the sheath part 3. This configuration can prevent the bleeding out of the insect repellent 4 to the outer surface of the insect repellent fiber 1 and the occurrence of the tackiness. As a result, when the insect repellent screen as a woven product is configured using the insect repellent fiber 1, the insect repellent fiber 1 can be easily woven.

Further, when the tackiness easily occurs on the surface of the insect repellent fiber, the dusts and the like tend to adhere to the surface of the insect repellent fiber. The dusts and the like adhering to the surface of the insect repellent fiber inhibit the release of the insect repellent from the insect repellent fiber and reduce the insect repellent effect, which is unfavorable. Further, when the insect repellent fiber is washed (for example, with water) to remove the dusts and the like adhering to the insect repellent fiber, the insect repellent is also removed along with the dusts and the like, which undesirably facilitates the release of the insect repellent from the insect repellent fiber and shortens the duration of the insect repellent effect.

On the other hand, the present embodiment can prevent the insect repellent 4 from bleeding out to the surface of the insect repellent fiber 1 as described above, thereby enabling to prevent the adhesion of the dusts and the like to the surface of the insect repellent fiber 1 and the inhibition of the release of the insect repellent 4 caused by the dusts and the like. Further, the present embodiment can also reduce the number of washing times of the insect repellent fiber 1, thereby enabling to prevent the removal of the insect repellent 4 caused by washing and prolong the duration of the insect repellent effect.

According to the insect repellent fiber 1 of the present embodiment, in which permethrin is used as the insect repellent 4, the amount of the insect repellent 4 (permethrin) can be set to 0.4 $\mu$g or more and 10 $\mu$g or less per gram of the insect repellent fiber 1, when the amount of the insect repellent 4 (permethrin) exposed to the surface of the insect repellent fiber 1 is measured by an acetone washing method. In the acetone washing method, the surface of the insect repellent fiber 1 is subjected to a washing treatment using an absorbent cotton wetted by an organic solvent such as acetone and the organic solvent such as acetone is extracted from the absorbent cotton used in the washing treatment to measure the amount of the insect repellent 4 contained in the organic solvent. The amount of the insect repellent 4 described above is calculated from the measurement result obtained by the acetone washing method and represents the amount of the insect repellent 4 per gram of the insect repellent fiber 1.

When the amount of the insect repellent 4 (permethrin) exposed to the surface of the insect repellent fiber 1 is less than 0.4 $\mu$g per gram of the insect repellent fiber 1, the insect repellent effect cannot be sufficiently exerted. On the other hand, when the amount of the insect repellent 4 (permethrin) is greater than 10 $\mu$g per gram of the insect repellent fiber 1, the insect repellent 4 released from the insect repellent fiber 1 may be ingested by people and animals that come into contact with the insect repellent fiber 1 in an amount exceeding the acceptable intake which does not cause an appreciable health risk. The WHO and FAO (Food and Agriculture Organization of the United Nations) have investigated harmful effects of permethrin to a human body on the basis of a large amount of data obtained by animal tests and the like. According to the investigation results, the amount of permethrin that can be ingested by human over a lifetime without an appreciable health risk, or also referred to as ADI (Acceptable Daily Intake), is set to "0.05 mg/day/kg". For example, for people who weigh 15 kg (weight of around a three-year-old child), this value per day is calculated to be 0.75 mg. This means that permethrin of such an amount may be continually ingested without an appreciable health risk. In the present embodiment, in which permethrin is used as the insect repellent 4, the (maximum) amount of the insect repellent 4 (permethrin) reaches 0.75 mg when 75 g of the insect repellent fiber 1 is used. In a product using the insect repellent fiber 1 (for example, a screen), the weight of the insect repellent fiber 1 rarely exceeds 75 g, thus using the product produced from the insect repellent fiber 1 is harmless to the health of a living being such as human.

Further, increasing the amount of insect repellent 4 causes the tackiness to be generated on the surface of the insect repellent fiber 1, thereby degrading weaving property and lowering the insect repellent effect by facilitating the adhesion of the dusts to the insect repellent fiber 1.

In the present embodiment, a cross-sectional shape of the sheath-core structure orthogonal to the longitudinal direction of the insect repellent fiber 1 is not particularly limited, but it is preferable that the cross section of the insect repellent fiber 1 have a circular shape and the core part 2 and the sheath part 3 be concentrically formed. The sheath part 3 has a thickness of preferably 5 μm or more and 70 μm or less. When the thickness of the sheath part 3 is more than 70 μm, as compared with the case where the thickness of the sheath part 3 is 70 μm or less, the insect repellent 4 hardly passes through the sheath part 3, thereby causing a reduction in the insect repellent effect of the insect repellent fiber 1. On the other hand, when the thickness of the sheath part 3 is less than 5 μm, as compared with the case where the thickness of the sheath part 3 is 5 μm or more, controlling the thickness becomes more difficult, thus forming the sheath part 3 becomes more difficult.

When the cross section of the insect repellent fiber 1 of the present embodiment has a circular shape, the diameter of the insect repellent fiber 1 may be appropriately set without particular limitation, however, it may be preferably set to 50 μm or more and 250 μm or less. Adjusting the diameter of the insect repellent fiber 1 to 50 μm or more and 250 μm or less enables to provide the insect repellent screen produced from the insect repellent fiber 1 with sufficient mechanical strength. When the diameter of the insect repellent fiber 1 is less than 50 μm, the production of the insect repellent fiber 1 having the sheath-core structure becomes more difficult and needs more manufacturing steps, which are not desirable from an aspect of manufacturing costs. Thus, the diameter of the insect repellent fiber 1 is preferably set to 50 μm or more for the production of the insect repellent fiber 1. On the other hand, when the diameter of the insect repellent fiber 1 is more than 250 μm, it becomes difficult to increase the number of meshes of the insect repellent screen when producing the insect repellent screen from the insect repellent fiber 1. As a result, the openings of the insect repellent screen tend to be larger and insects are more likely to intrude from the openings of the insect repellent screen. Thus, the diameter of the insect repellent fiber 1 is preferably set to 250 μm or less from the viewpoint of maintaining the sufficient number of meshes of the insect repellent screen.

When the cross section of the insect repellent fiber 1 of the present embodiment has a circular shape, the diameter of the insect repellent fiber 1, the diameter of the core part 2, and the thickness of the sheath part 3 can be measured, for example, using a microscope .

A production method of the insect repellent fiber 1 according to the present embodiment may be appropriately selected by a person skilled in the art without particular limitation. For example, the insect repellent fiber 1 can be produced in a reasonable and low-cost manner by filling the thermoplastic resin configuring the core part 2 of the insect repellent fiber 1 with the insect repellent 4 and then producing the core part 2 and the sheath part 3 by the melt spinning. Specifically, masterbatch pellets are produced in advance using pellets of the thermoplastic resin or the like containing the insect repellent 4 in liquid form. The core part 2 is produced by mixing the masterbatch pellets with pellets of the same thermoplastic resin as used for the masterbatch pellets by a predetermined ratio. Then, the insect repellent fiber 1 of the present embodiment is produced by a known core-sheath spinning device. In the core-sheath spinning device, the pellets of the thermoplastic resin are used for the sheath part 3.

In this method, the insect repellent fiber 1 may be subjected to the heating and stretching process after the spinning. In this process, the crystallinity of the thermoplastic resin, the outer diameter of the insect repellent fiber 1, and the like can be controlled by adjusting the heating temperature and stretch ratio in the heating and stretching process.

When the core part 2 is produced by filling the thermoplastic resin with the insect repellent 4 in liquid form, the insect repellent 4 may be evenly dispersed in the core part 2. However, in another form of the core part 2, the insect repellent 4 may be unevenly present in the core part 2. For example, as shown in FIG.2, the insect repellent 4 may be filled in only one side half of the cross section orthogonal to the longitudinal direction of the core part 2. For example, when the insect repellent fiber 1 including such a core part 2 is used as an agricultural insect repellent screen, the insect repellent 4 can be unevenly distributed to one side of the insect repellent screen. When crops are covered with the insect repellent screen, one side of the insect repellent screen free from the insect repellent 4 can be placed on the side of the crops and the other side of the insect repellent screen where the insect repellent 4 exists can be placed on the side opposite to the crop side. In this manner, the insect repellent 4, which are released to the side opposite to the crop side, can prevent insects from coming close to the insect repellent screen. Further, the insect repellent 4 is hardly released toward the crops, and thus adhesion of the insect repellent 4 to the crops can be prevented.

In the present embodiment, the content of the insect repellent 4 in the insect repellent fiber 1 is preferably 0.1% by mass or more and 10% by mass or less with respect to the insect repellent fiber 1. When the content of the insect repellent 4 is less than 0.1% by mass, as compared with the case where the content of the insect repellent 4 is 0.1% by mass or more, the insect repellent effect decreases and the duration of the insect repellent effect is shortened. When the content of the insect repellent 4 is more than 10% by mass, as compared with the case where the content of the insect repellent 4 is 10% by mass or less, the mass percent of the resins configuring the core part 2 and the sheath part 3 serving as a skeleton of the insect repellent fiber 1 decreases, thereby causing a reduction in strength of the insect repellent fiber 1. Further, this causes the tackiness and makes the weaving difficult. Further, although it depends on the thickness of the sheath part 3, the exposure amount of the insect repellent 4 on the surface of the insect repellent fiber 1 is increased as the content of the insect repellent 4 becomes larger. As a result, the intake of the insect repellent 4 by people and animals is increased. The content of the insect repellent 4 in the insect repellent fiber 1 is more preferably 0.1% by mass or more and 5% by mass or less with respect to the insect repellent fiber 1. Adjusting the content of the insect repellent 4 to 0.1% by mass or more and 5% by mass or less can prevent the occurrence of the tackiness and defective weaving. Further, this content can prevent the exposure of the insect repellent 4 and secure the safety for a living being such as human.

Further, the insect repellent fiber 1 of the present embodiment may include a component for imparting an optional function as a functional material. Examples of the functional material may include titanium dioxide as a delustering agent, calcium stearate as a lubricant, micro particles of silica, alumina, or the like, a hindered phenol derivative as an antioxidant, and additive materials such as a coloring agent including a pigment or the like, a stabilizer, and a dispersant. Further examples of the functional material may include an ultraviolet ray shielding agent, a near-infrared ray shielding agent, an antibacterial agent, an antifungal agent, an antistatic agent, a flame retardant, a weathering agent, and various kinds of catalysts. It is noted that the functional material may be dispersed in the core part 2 together with the insect repellent 4, the functional material may be dispersed in the sheath part 3, or the functional material may adhere to the surface of the insect repellent fiber 1.

Further, inorganic fine particles may be chemically bonded to the surface of the insect repellent fiber 1 according to the present embodiment to form fine irregularities. Forming the fine irregularities further prevents the adhesion of the dusts and the like floating in the air to the surface of the insect repellent fiber 1. Further, the dust and the like adhering to the surface of the insect repellent fiber 1 can be easily washed off by water or the like without removing the insect repellent 4 exposed to the surface of the insect repellent fiber 1. Thus, the insect repellent fiber 1 can exhibit excellent dustproof property.

In the present embodiment, the cross section of the insect repellent fiber 1 may have an irregular shape, such as circular, flat, triangular, hollow, or star-shaped. Among the cross-sectional shapes described above, the cross section of the insect repellent fiber 1 preferably has the circular shape from the viewpoint of wear resistance, attitude stability, and smoothness. Further, the monofilament shown in FIG. 1 or multifilament may be appropriately selected to form the insect repellent fiber 1 in accordance with the purpose of use.

Further, the insect repellent fiber 1 may have a hollow part or the insect repellent fiber 1 may be formed as a composite fiber, such as a sea-island type composite fiber. The insect repellent fiber 1 having the hollow part may be formed in a multilayer structure . In this structure, an inner layer forming the hollow part may be formed from the same material as that of the core part 2 of the present embodiment. Further, an outer layer formed outside the inner layer may be formed from the same material as that of the sheath part 3 of the present embodiment. When the insect repellent fiber 1 is formed as the sea-island type composite fiber, island parts may be formed from the same material as that of the core part 2 of the present embodiment, while a sea part may be formed from the same material as that of the sheath part 3 of the present embodiment.

The insect repellent fiber 1 of the present embodiment may be used, for example, as warp fibers (lengthwise fibers) and/or weft fibers (crosswise fibers) . These fibers may be woven by a known method to obtain the insect repellent screen configured in a mesh structure having substantially rectangular openings. Further, the mesh structure of the insect repellent screen may be subjected to the pleating process in the lateral or longitudinal direction.

The width and height of the openings of the mesh structure may be appropriately set without particular limitation. However, the width and height are each preferably 100 $\mu$m or more and 1,500 $\mu$m or less. When at least one of the width and height of the openings is less than 100 $\mu$m, it becomes difficult to obtain a configuration having a large opening rate, and thus the air permeability of the mesh structure is reduced. When at least one of the width and height of the openings is more than 1, 500 $\mu$m, the air permeability of the mesh structure can be sufficiently maintained, however, small insects can easily pass through the openings of the mesh structure.

The insect repellent fiber 1 according to the present embodiment thus obtained can release the minimum amount of the insect repellent 4 required to exhibit the insect repellent effect and can easily secure the duration of the insect repellent effect. The insect repellent fiber 1 of the present embodiment can be used in various applications, such as for an agricultural insect repellent screen, a household screen door, an accordion screen door, and a mosquito net.

[EXAMPLES]

Hereinafter, the insect repellent fiber and the insect repellent screen of the present embodiment will be described in more details with reference to Examples. However, a technical scope of the present invention is not limited to these Examples.

(Example 1)

Masterbatch pellets formed from a highly crystalline homo polypropylene resin containing permethrin (an insect repellent) were prepared. Pellets formed from the highly crystalline homo polypropylene resin were prepared. The masterbatch pellets and the highly crystalline homo polypropylene resin were melted and mixed to obtain a mixture containing permethrin in a predetermined amount. The obtained mixture and the prepared pellets were separately melted using a melting extruder equipped in a melt spinning machine. The melted mixture and pellets were ejected from a spinneret for core-sheath type composite fiber equipped in the melt spinning machine, and the ejected product was taken up at a predetermined take-up speed while being cooled in a water tank to obtain a fiber. The fiber obtained continuously was stretched at a predetermined stretch ratio while being passed through warm water (a stretching tank) heated to a predetermined temperature. The stretched fiber was wound around a bobbin while being passed through a setting tank to obtain an insect repellent fiber. The insect repellent fiber was formed in a monofilament having a sheath-core structure including a core part (diameter of core part: 194 $\mu$m) formed from the highly crystalline homo polypropylene resin containing permethrin and a sheath part (thickness of sheath part: 28 $\mu$m) formed from the highly crystalline homo polypropylene resin. The content of permethrin with respect to the obtained insect repellent fiber was 1% by mass. The insect repellent fibers were subjected to weaving using a conventional Sulzer loom (manufactured by Sulzer Ltd.) to obtain an insect repellent screen. The insect repellent screen was plain-woven fabric having both density of warp fibers and weft fibers of 20/2.54cm. This insect repellent screen was defined as the insect repellent screen of Example 1.

(Example 2)

Masterbatch pellets formed from a highly crystalline homo polypropylene resin containing ethofenprox were used instead of the masterbatch pellets used in Example 1. Other than that, the same conditions as those of Example 1 were used to obtain an insect repellent fiber. The insect repellent fiber was formed in a monofilament having a sheath-core structure including a core part (diameter of core part: 194 $\mu$m) formed from the highly crystalline homo polypropylene resin containing ethofenprox and a sheath part (thickness of sheath part: 28 $\mu$m) formed from the highly crystalline homo polypropylene resin. The content of ethofenprox with respect to the obtained insect repellent fiber was 1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 2.

(Example 3)

Pellets formed from a low crystalline random polypropylene resin were used instead of the pellets formed from the highly crystalline homo polypropylene resin used in Example 1. Other than that, the same conditions as those of Example 1 were used to obtain an insect repellent fiber. The insect repellent fiber was formed in a monofilament having a sheath-core structure including a core part (diameter of core part: 194 $\mu$m) formed from the highly crystalline homo polypropylene resin containing permethrin and a sheath part (thickness of sheath part: 28 $\mu$m) formed from the low crystalline random polypropylene resin. The content of permethrin with respect to the obtained insect repellent fiber was 1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 3.

(Example 4)

A mixture used in this Example had a different permethrin content from the mixture used in Example 1. This Example also used a different spinneret for core-sheath type composite fiber from the one used in Example 1. Other than that, the same conditions as those of Example 1 were used to obtain an insect repellent fiber. The insect repellent fiber was formed in a monofilament having a sheath-core structure including a core part (diameter of core part: 240 $\mu$m) formed from the highly crystalline homo polypropylene resin containing permethrin and a sheath part (thickness of sheath part: 5 $\mu$m) formed from the highly crystalline homo polypropylene resin. The content of permethrin with respect to the obtained insect repellent fiber was 1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 4.

(Example 5)

A mixture used in this Example had a different permethrin content from the mixture used in Example 1. This Example also used a different spinneret for core-sheath type composite fiber from the one used in Example 1. Other than that, the same conditions as those of Example 1 were used to obtain an insect repellent fiber. The insect repellent fiber was formed in a monofilament having a sheath-core structure including a core part (diameter of core part: 110 μm) formed from the highly crystalline homo polypropylene resin containing permethrin and a sheath part (thickness of sheath part: 70 μm) formed from the highly crystalline homo polypropylene resin. The content of permethrin with respect to the obtained insect repellent fiber was 1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 5.

(Example 6)

A mixture used in this Example had a different permethrin content from the mixture used in Example 1. This Example also used a different spinneret for core-sheath type composite fiber from the one used in Example 1. Other than that, the same conditions as those of Example 1 were used to obtain an insect repellent fiber. The insect repellent fiber was formed in a monofilament having a sheath-core structure including a core part (diameter of core part: 90 μm) formed from the highly crystalline homo polypropylene resin containing permethrin and a sheath part (thickness of sheath part: 80 μm) formed from the highly crystalline homo polypropylene resin. The content of permethrin with respect to the obtained insect repellent fiber was 1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 6.

(Example 7)

A mixture used in this Example had a different permethrin content from the mixture used in Example 1. Other than that, the same conditions as those of Example 1 were used to obtain an insect repellent fiber. The insect repellent fiber was formed in a monofilament having a sheath-core structure including a core part (diameter of core part: 194 μm) formed from the highly crystalline homo polypropylene resin containing permethrin and a sheath part (thickness of sheath part: 28 μm) formed from the highly crystalline homo polypropylene resin. The content of permethrin with respect to the obtained insect repellent fiber was 3% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 7.

(Example 8)

A mixture used in this Example had a different permethrin content from the mixture used in Example 1. Other than that, the same conditions as those of Example 1 were used to obtain an insect repellent fiber. The insect repellent fiber was formed in a monofilament having a sheath-core structure including a core part (diameter of core part: 194 μm) formed from the highly crystalline homo polypropylene resin containing permethrin and a sheath part (thickness of sheath part: 28 μm) formed from the highly crystalline homo polypropylene resin. The content of permethrin with respect to the obtained insect repellent fiber was 0.1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 8.

(Example 9)

A mixture used in this Example had a different permethrin content from the mixture used in Example 1. Other than that, the same conditions as those of Example 1 were used to obtain an insect repellent fiber. The insect repellent fiber was formed in a monofilament having a sheath-core structure including a core part (diameter of core part: 194 μm) formed from the highly crystalline homo polypropylene resin containing permethrin and a sheath part (thickness of sheath part: 28 μm) formed from the highly crystalline homo polypropylene resin. The content of permethrin with respect to the obtained insect repellent fiber was 5% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 9.

(Example 10)

A mixture used in this Example had a different permethrin content from the mixture used in Example 1. Other than that, the same conditions as those of Example 1 were used to obtain an insect repellent fiber. The insect repellent fiber was formed in a monofilament having a sheath-core structure including a core part (diameter of core part: 194 $\mu$m) formed from the highly crystalline homo polypropylene resin containing permethrin and a sheath part (thickness of sheath part: 28 $\mu$m) formed from the highly crystalline homo polypropylene resin. The content of permethrin with respect to the obtained insect repellent fiber was 0.01% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Example 10.

(Comparative example 1)

The masterbatch pellets and the pellets used in Example 1 were melted and mixed to obtain a mixture containing permethrin in a predetermined content. The obtained mixture was melted and ejected from a spinneret equipped in the melt spinning machine. Other than that, the same conditions as those of Example 1 were used to obtain an insect repellent fiber (diameter of fiber of 250 $\mu$m). The insect repellent fiber was formed in a monofilament of a single layer structure formed from permethrin and the highly crystalline homo polypropylene resin. The content of permethrin with respect to the obtained insect repellent fiber was 1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Comparative example 1.

(Comparative example 2)

The masterbatch pellets and the pellets used in Example 2 were melted and mixed to obtain a mixture containing ethofenprox in a predetermined content. The obtained mixture was melted and ejected from a spinneret equipped in the melt spinning machine. Other than that, the same conditions as those of Example 2 were used to obtain an insect repellent fiber (diameter of fiber of 250 $\mu$m). The insect repellent fiber was formed in a monofilament of a single layer structure formed from ethofenprox and the highly crystalline homo polypropylene resin. The content of ethofenprox with respect to the obtained insect repellent fiber was 1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Comparative example 2.

(Comparative example 3)

The pellets used in Example 1 were melted. The melted product thus obtained was ejected from a spinneret equipped in the melt spinning machine . Other than that, the same conditions as those of Example 1 were used to obtain an insect repellent fiber (diameter of fiber of 250 $\mu$m). The insect repellent fiber was formed in a monofilament of a single layer structure formed from the highly crystalline homo polypropylene resin. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Comparative example 3.

(Comparative example 4)

Masterbatch pellets formed from the low crystalline random polypropylene resin containing permethrin were prepared. Pellets formed from the highly crystalline homo polypropylene resin were prepared. The masterbatch pellets and the low crystalline homo polypropylene resin were melted and mixed to obtain a mixture containing permethrin in a predetermined content. The obtained mixture and the prepared pellets were separately melted using the melting extruder equipped in the melt spinning machine. The melted mixture and the pellets were ejected from a spinneret for core-sheath type composite fiber equipped in the melt spinning machine, and the ejected product was taken up at a predetermined take-up speed while being cooled in the water tank to obtain a fiber. The fiber obtained continuously was stretched at a predetermined stretch ratio while being passed through warm water (a stretching tank) heated to a predetermined temperature. The stretched fiber was wound around a bobbin while being passed through the setting tank to obtain an insect repellent fiber. The insect repellent fiber was formed in a monofilament having a sheath-core structure including a core part (diameter of core part: 194 μm) formed from the low crystalline random polypropylene resin containing permethrin and a sheath part (thickness of sheath part: 28 μm) formed from the highly crystalline homo polypropylene resin. The content of permethrin with respect to the obtained insect repellent fiber was 1% by mass. The insect repellent fibers were subjected to weaving using the conventional Sulzer loom to obtain an insect repellent screen. The insect repellent screen was plain-woven fabric having both density in warp fibers and weft fibers of 20/2.54cm. This insect repellent screen was defined as the insect repellent screen of Comparative example 4.

(Comparative example 5)

A mixture used in this Example had a different permethrin content from the mixture used in Example 1. The materials used in the core part and the sheath part in Example 1 were switched. Other than that, the same conditions as those of Example 1 were used to obtain an insect repellent fiber. The insect repellent fiber was formed in a monofilament having a sheath-core structure including a core part (diameter of core part: 194 μm) formed from the highly crystalline homo polypropylene resin and a sheath part (thickness of sheath part: 28 μm) formed from the highly crystalline homo polypropylene resin containing permethrin. The content of permethrin with respect to the obtained insect repellent fiber was 1% by mass. The insect repellent fibers were subjected to weaving using the conventional Sulzer loom to obtain an insect repellent screen. The insect repellent screen was plain-woven fabric having both density of warp fibers and weft fibers of 20/2.54cm. This insect repellent screen was defined as the insect repellent screen of Comparative example 5.

(Evaluation of crystallinity by measurement)

The crystallinity of the polypropylene resins configuring the sheath parts of the insect repellent screens produced in Examples 1 to 10 and Comparative examples 1 to 5 (crystallinity of the polypropylene resins configuring the insect repellent screens for Comparative examples 1 to 3) was measured by the powder X-ray diffraction method. Further, the crystallinity of the polypropylene resins configuring the core parts was obtained by measuring, by the powder X-ray diffraction method, the crystallinity of the polypropylene resins configuring core parts formed in single layer structures(i.e. fiber) under the same conditions as those for the corresponding core parts. Results are shown in Table 1.

[Table 1]

| | | | INSECT REPELLENT | | INSECT REPELLENT CONTENT(%) | SHEATH THICKNESS (μm) | CRYSTALLINITY (%) | |
| --- | --- | --- | --- | --- | --- | --- | --- | --- |
| | | | CORE PART | SHEATH PART | | | CORE PART | SHEATH PART |
| EXAMPLES | | 1 | PERMETHRIN | - | 1 | 28 | 83 | 80 |
| | | 2 | ETHOFENPROX | - | 1 | 28 | 83 | 80 |
| | | 3 | PERMETHRIN | - | 1 | 28 | 83 | 68 |
| | | 4 | PERMETHRIN | - | 1 | 5 | 83 | 80 |
| | | 5 | PERMETHRIN | - | 1 | 70 | 83 | 80 |
| | | 6 | PERMETHRIN | - | 1 | 80 | 83 | 80 |
| | | 7 | PERMETHRIN | - | 3 | 28 | 83 | 80 |
| | | 8 | PERMETHRIN | - | 0.1 | 28 | 83 | 80 |
| | | 9 | PERMETHRIN | - | 5 | 28 | 83 | 80 |
| | | 10 | PERMETHRIN | - | 0.01 | 28 | 83 | 80 |
| COMPARATIVE EXAMPLES | | 1 | PERMETHRIN | | 1 | - | 83 | |
| | | 2 | ETHOFENPROX | | 1 | - | 83 | |
| | | 3 | - | | - | - | 80 | |
| | | 4 | PERMETHRIN | - | 1 | 28 | 68 | 83 |
| | | 5 | - | PERMETHRIN | 1 | 28 | 83 | 80 |

(Evaluation 1: evaluation of safety)

In order to evaluate the safety of the insect repellent fibers and the insect repellent screens of the present embodiment, the following evaluation test was performed.

The surfaces of the insect repellent screens of Examples 1 to 10 and Comparative examples 1 to 5 were wiped with absorbent cottons wetted by acetone and the acetone was extracted from the absorbent cottons. In each sample, the amount of the insect repellent contained in the extracted acetone was measured by the gas chromatograph mass spectrometer (GC-MS) to calculate the amount of the insect repellent ($\mu$g/g) exposed to the surface of the insect repellent fiber per gram thereof . On the basis of calculation results, the amount of the insect repellent (mg) exposed to the surface of the insect repellent fiber per square meter thereof was calculated in each sample.

The safety was evaluated according to the following evaluation criteria by assuming a situation that a child who weighs 15 kg licked a whole area of a screen door in which the insect repellent screen having an area of 1 m$^2$ was installed. It is noted that the acceptable intake of the insect repellent used in the evaluation criteria is 0.75 mg for permethrin and 0.45 mg for ethofenprox. It is noted that when the insect repellent screen was evaluated as "good", the insect repellent screen was determined to be safe. Results are shown in Table 2 below.

[Evaluation criteria]

Good: the amount of the insect repellent exposed to the surface of the insect repellent screen per square meter thereof is less than or equal to the acceptable intake per day calculated on the basis of AID for a child who weighs 15 kg.
Poor: the amount of the insect repellent exposed to the surface of the insect repellent screen per square meter thereof is greater than the acceptable intake per day calculated on the basis of AID for a child who weighs 15 kg.

(Evaluation 2: evaluation of repelling property)

In order to evaluate repelling property of the insect repellent fibers and the insect repellent screens of the present embodiment, the following evaluation test was performed.

The insect repellent screens of Examples 1 to 10 and Comparative examples 1 to 5 were cut into a predetermined size. After cut, the insect repellent screens of Examples 1 to 10 and Comparative examples 1 to 5 were used to cover mice. The covered mice represented specimens for Examples 1 to 10 and Comparative examples 1 to 5 as follows.

Specimen of Example 1: mouse covered with the insect repellent screen of Examples 1.
Specimen of Example 2: mouse covered with the insect repellent screen of Examples 2.
Specimen of Example 3: mouse covered with the insect repellent screen of Examples 3.
Specimen of Example 4: mouse covered with the insect repellent screen of Examples 4.
Specimen of Example 5: mouse covered with the insect repellent screen of Examples 5.
Specimen of Example 6: mouse covered with the insect repellent screen of Examples 6.
Specimen of Example 7: mouse covered with the insect repellent screen of Examples 7.
Specimen of Example 8: mouse covered with the insect repellent screen of Examples 8.
Specimen of Example 9: mouse covered with the insect repellent screen of Examples 9.
Specimen of Example 10: mouse covered with the insect repellent screen of Examples 10.
Specimen of Comparative example 1: mouse covered with the insect repellent screen of Comparative example 1.
Specimen of Comparative example 2: mouse covered with the insect repellent screen of Comparative example 2.
Specimen of Comparative example 3: mouse covered with the insect repellent screen of Comparative example 3.
Specimen of Comparative example 4: mouse covered with the insect repellent screen of Comparative example 4.
Specimen of Comparative example 5: mouse covered with the insect repellent screen of Comparative example 5.

In an acrylic resin box (30-centimeter cube), 20 non-blood sucking female imagoes of Aedes albopictus were released, and each of the specimens of the Examples 1 to 10 and Comparative examples 1 to 5 was placed in the acrylic resin box. The number of landing of Aedes albopictus on each specimen (hereinafter also referred to as "the landing number") was counted for 30 seconds after the specimen was placed. The counting was continued for 5 minutes.

The total landing number for each specimen for 5 minutes after the specimen was placed was calculated on the basis of the counting results described above. The repelling rate of each insect repellent screen covering the corresponding specimen was calculated on the basis of the following formula (a) using the total landing number calculated for the specimen, and the total landing number obtained from the specimen of Comparative example 3, in which the mouse was covered with the insect repellent screen containing no insect repellents, as a reference.

$$(X - Y)/X \times 100 \quad \dots \text{(a)}$$

In the formula (a), X represents the total landing number obtained from the specimen of Comparative example 3 and Y represents the total landing number obtained from each specimen.

The repel ling property was evaluated according to the following evaluation criteria. It is noted that, when the insect repellent screen was evaluated as "A", it was determined to have excellent repelling property, when evaluated as "B", it was determined to have repelling property, and when evaluated as "C", it was determined to have no repelling property. Results are shown in Table 2 below.

[Evaluation criteria]

A: repelling rate of more than 60%
B: repelling rate of 30 to 60%
C: repelling rate of less than 30%

(Evaluation 3 : evaluation of dust adhesion restraining effect)

In order to evaluate dust adhesion restraining effect of the insect repellent fibers and the insect repellent screens of the present embodiment, the following evaluation test was performed.

The insect repellent screens of Examples 1 to 10 and Comparative examples 1 to 5 were cut into 10-centimeter square, and mixed dusts were uniformly placed on the respective upper surfaces of the insect repellent screens after cut. The insect repellent screens on which the mixed dusts were placed were lifted up, flipped over, and imparted with predetermined times of vibration to drop down the mixed dusts that did not adhere to the insect repellent screens. The weight of the mixed dusts that adhered to the insect repellent screens was calculated by measuring the weight of the insect repellent screens before the mixed dusts were placed and the weight of the insect repellent screens after the unadhering mixed dusts were dropped. The average weight of the mixed dusts that adhered to the insect repellent screens was calculated by repeating this operation three times . It is noted that fifteen kinds of particles of Test Powders 1 specified in JIS Z 8901 were used as the mixed dusts.

The dust adhesion restraining effect was evaluated by the following evaluation criteria. It is noted that when the insect repellent screen was evaluated as "Good", it was determined to have the dust adhesion restraining effect. Results are shown in Table 2 below.

[Evaluation criteria]

Good: the average weight of the mixed dusts adhering to the insect repellent screen is 100 mg or less.

Poor: the average weight of the mixed dusts adhering to the insect repellent screen is more than 100 mg.

[Table 2]

| | | EVALUATION 1 | | EVALUATION 2 | | EVALUATION 3 | |
|---|---|---|---|---|---|---|---|
| | | AMOUNT OF INSECT REPELLENTS EXPOSED TO 1m$^2$ OF INSECT REPELLENT SCREEN (mg) | EVALUATION RESULTS | REPELLING (%) | EVALUATION RESULTS | ADHESION AMOUNT OF MIXED DUSTS (mg) | EVALUATION RESULTS |
| EXAMPLES | 1 | 0.204 | Good | 92 | A | 42 | Good |
| | 2 | 0.340 | Good | 87 | A | 42 | Good |
| | 3 | 0.408 | Good | 92 | A | 60 | Good |
| | 4 | 0.340 | Good | 100 | A | 58 | Good |
| | 5 | 0.058 | Good | 68 | A | 38 | Good |
| | 6 | 0.007 | Good | 42 | B | 35 | Good |
| | 7 | 0.476 | Good | 100 | A | 57 | Good |
| | 8 | 0.041 | Good | 64 | A | 38 | Good |
| | 9 | 0.748 | Good | 100 | A | 94 | Good |
| | 10 | 0.005 | Good | 34 | B | 32 | Good |
| COMPARATIVE EXAMPLES | 1 | 3.604 | Poor | 100 | A | 108 | Poor |
| | 2 | 8.160 | Poor | 100 | A | 120 | Poor |
| | 3 | - | - | 0 | C | 27 | Good |
| | 4 | 0.001 | Good | 28 | C | 31 | Good |
| | 5 | 3.468 | Poor | 100 | A | 106 | Poor |

As shown in Table 2, the insect repellent screens of Examples 1 to 10 were evaluated as "Good" in the evaluation 1 (evaluation of safety) and evaluation 3 (evaluation of dust adhesion restraining effect), and evaluated as "A" or "B" in the evaluation 2 (evaluation of repelling property) . As is clear from the results, the insect repellent screens of the present embodiment are safe and have the repelling property and the dust adhesion restraining effect. More specifically, the insect repellent fibers of the present embodiment configured as described above can control the amount of the insect repellent exposed to the surface of the insect repellent fiber to the minimum amount necessary to exert the insect repellent effect, thus enabling to secure the safety and preventing the adhesion of the dusts. Further, the insect repellent screens of Examples 1 to 10 had the dust adhesion restraining effect. The results indicates that the insect repellent screens of the present embodiment can prolong the insect repellent effect, the insect repellent screens of the present embodiment can prevent the occurrence of the tackiness (stickiness), and the insect repellent screens of the present embodiment can allow easy weaving.

On the other hand, the insect repellent screens of Comparative examples 1 to 5 were evaluated as "Poor" or "C" in at least one of the evaluations 1 to 3. The results show that the insect repellent screens of Comparative examples 1 to 5 fail to achieve at least one of the safety, the repelling property, and the dust adhesion restraining effect.

In particular, the insect repellent screens of Comparative examples 1, 2, and 5 were evaluated as "Poor" in the evaluation 1. The results show that the insect repellent is easily exposed to the surface of the insect repellent fiber in the insect repellent fiber formed in the single layer structure and the insect repellent fiber having the sheath-core structure in which the insect repellent is contained in the sheath part. Further, the insect repellent screens of Comparative examples 1, 2 and 5 were evaluated as "Poor" in the evaluation 3. As one of the factors contributing to such a result, it is conceivable that the insect repellent fiber formed in the single layer structure and the insect repellent fiber having the sheath-core structure in which the insect repellent is contained in the sheath part facilitate the exposure of the insect repellent to the surface of the insect repellent fiber and the adhesion of the dusts is facilitated.

Further, the insect repellent screen of Comparative example 4 was evaluated as "C" in the evaluation 2, and, as shown in the results of the evaluation 1, the amount of the insect repellent exposed to the surface was less than those of the insect repellent screens of Examples 1 to 10. The results show that, in the insect repellent fiber in which the crystallinity of the thermoplastic resin configuring the sheath part is larger than the crystallinity of the thermoplastic resin configuring the core part, the insect repellent hardly migrates in the sheath part to be released to the outside of the insect repellent fiber.

It is noted that the insect repellent screen of Comparative example 3 had the smallest amount of the adhering mixed dusts in the evaluation 3. This may be because of the absence of the insect repellent in the insect repellent fiber.

Further, the insect repellent screens of Examples 1 to 9, in which the content of the insect repellent with respect to the insect repellent fiber is 0.1% by mass or more, improved the repelling rate by 8% or more as compared with the insect repellent screen of Example 10, in which the content of the insect repellent is less than 0.1% by mass (0.01% by mass) . The results show that the insect repellent fiber having the content of the insect repellent of 0.1% by mass or more can further facilitate the exertion of the insect repellent effect as compared with the insect repellent fiber having the content of the insect repellent of less than 0.1% by mass.

It is noted that the acceptable intake of permethrin (0.75 mg) in the evaluation 1 corresponds to the amount of permethrin exposed to the surface of the insect repellent screen having an area of 1.30 $m^2$ in Example 1. On the other hand, the acceptable intake of ethofenprox (0.45 mg) in the evaluation 1 corresponds to the amount of ethofenprox exposed to the surface of the insect repellent screen having an area of 0.053 $m^2$ in Comparative example 2. That is, in the insect repellent screen of Example 1, even if a child who weighs 15 kg accidentally licks a whole area of one screen door (the insect repellent screen having an area of 1 $m^2$), the intake does not exceed the acceptable intake described above. However, in the insect repellent screen of Comparative example 2, the intake reaches the acceptable intake described above by licking about one twentieth of the screen door (the insect repellent screen having an area of 0.053 $m^2$). This shows that the insect repellent screen of Example 1 is excellent in the safety.

Reference Signs List

1:    insect repellent fiber
2:    core part
3:    sheath part
4:    insect repellent

**Claims**

1. An insect repellent fiber capable of releasing an insect repellent, comprising:

   a core part containing the insect repellent and being formed from a thermoplastic resin; and
   a sheath part being in contact with an outer surface of the core part to cover the core part and being formed from a thermoplastic resin, wherein
   the thermoplastic resin configuring the sheath part has a crystallinity less than or equal to a crystallinity of the thermoplastic resin configuring the core part.

2. The insect repellent fiber according to claim 1, wherein a main component of the insect repellent is a pyrethroid-based insect repellent.

3. The insect repellent fiber according to claim 2, wherein the pyrethroid-based insect repellent is at least one or more kinds of permethrin and ethofenprox.

4. The insect repellent fiber according to any one of claims 1 to 3, wherein the crystallinity of the thermoplastic resin configuring the sheath part is 80% or less.

5. The insect repellent fiber according to any one of claims 1 to 4, wherein the sheath part has a thickness of 5 $\mu$m or more and 70 $\mu$m or less.

6. The insect repellent fiber according to any one of claims 1 to 5, wherein a content of the insect repellent with respect to the insect repellent fiber is 0.1% by mass or more and 5% by mass or less.

7. An insect repellent screen comprising the insect repellent fiber according to any one of claims 1 to 6.

1

FIG.2

## INTERNATIONAL SEARCH REPORT

| | International application No. |
| --- | --- |
| | PCT/JP2016/057317 |

A. CLASSIFICATION OF SUBJECT MATTER
*D01F8/04*(2006.01)i, *A01N25/34*(2006.01)i, *A01N53/08*(2006.01)i, *A01P7/04* (2006.01)i

According to International Patent Classification (IPC) or to both national classification and IPC

B. FIELDS SEARCHED

Minimum documentation searched (classification system followed by classification symbols)
D01F8/00-8/18, A01N25/34, A01N53/08, A01P7/04

Documentation searched other than minimum documentation to the extent that such documents are included in the fields searched
Jitsuyo Shinan Koho        1922-1996   Jitsuyo Shinan Toroku Koho   1996-2016
Kokai Jitsuyo Shinan Koho   1971-2016   Toroku Jitsuyo Shinan Koho   1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 8-134720 A  (Teijin Ltd.),<br>28 May 1996 (28.05.1996),<br>claims; paragraphs [0009], [0011], [0021]<br>(Family: none) | 1-6<br>2-3,7 |
| X<br>Y | JP 2000-248466 A  (Kuraray Co., Ltd.),<br>12 September 2000 (12.09.2000),<br>claims; paragraphs [0009], [0025]<br>(Family: none) | 1,4-6<br>2-3,7 |
| X<br>Y | JP 6-272112 A  (Mitsubishi Rayon Co., Ltd.),<br>27 September 1994 (27.09.1994),<br>claims; paragraphs [0013], [0017]<br>(Family: none) | 1,4-6<br>2-3,7 |

| [X] | Further documents are listed in the continuation of Box C. | | [ ] | See patent family annex. |
| --- | --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    01 June 2016 (01.06.16) | Date of mailing of the international search report<br>    14 June 2016 (14.06.16) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

**INTERNATIONAL SEARCH REPORT**

| International application No. |
|---|
| PCT/JP2016/057317 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2010-532325 A  (Vestergaard Frandsen S.A.), 07 October 2010 (07.10.2010), claims; paragraphs [0001], [0039] & US 2010/0136076 A1 claims; paragraphs [0001], [0039] & WO 2009/003468 A1     & EP 2170048 A & KR 10-2010-0051635 A  & CN 101720185 A & TW 200909635 A        & CN 105076131 A | 2-3,7 |
| Y | JP 2010-13390 A  (Sumitomo Chemical Co., Ltd.), 21 January 2010 (21.01.2010), claims; paragraph [0002] (Family: none) | 7 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010013390 A

- JP 2001220970 A